# EUROPÄISCHE PATENTSCHRIFT

(11) **EP 2 699 281 B1**
(45) Veröffentlichungstag und Bekanntmachung des Hinweises auf die Patenterteilung: **03.06.2015**
(21) Anmeldenummer: 12715606.5
(22) Anmeldetag: 12.04.2012
(51) Int. Cl.: A61M 1/34, A61M 1/36

(54) **VORRICHTUNG ZUR EXTRAKORPORALEN BLUTBEHANDLUNG UND VERFAHREN ZUR ÜBERWACHUNG DER FLÜSSIGKEITSSTRÖMUNG EINER EXTRAKORPORALEN BLUTBEHANDLUNGSVORRICHTUNG**
DEVICE FOR EXTRACORPOREAL BLOOD TREATMENT AND METHOD FOR MONITORING THE FLUID FLOW OF AN EXTRACORPOREAL BLOOD TREATMENT DEVICE
DISPOSITIF POUR LE TRAITEMENT EXTRACORPOREL DU SANG ET PROCÉDÉ POUR LA SURVEILLANCE D'UN FLUX DE LIQUIDE DANS UN DISPOSITIF DE TRAITEMENT EXTRACORPOREL DU SANG

(30) Priorität: 21.04.2011 DE 102011018601; 21.04.2011 US 201161477655 P
(43) Veröffentlichungstag der Anmeldung: 26.02.2014
(73) Patentinhaber: Fresenius Medical Care Deutschland GmbH, 61352 Bad Homburg v.d.H. (DE)
(72) Erfinder: HOCHREIN, Torsten, 97478 Knetzgau (DE); WERNER, Pascal, 97532 Üchtelhausen (DE); KIPP, Sabine, 61348 Bad Homburg (DE)
(74) Vertreter: Oppermann, Frank
(86) Internationale Anmeldenummer: PCT/EP2012/001594
(87) Internationale Veröffentlichungsnummer: WO 2012/143103

(56) Entgegenhaltungen:
- WO-A1-2006/050970
- WO-A2-91/15253
- DE-C1- 4 338 858
- US-A1- 2004 182 783
- US-A1- 2004 182 787
- US-A1- 2011 040 228

## Beschreibung

Die Erfindung betrifft eine Vorrichtung zur extrakorporalen Blutbehandlung, die über eine Einheit zur Gewinnung eines oder mehrerer Blutbestandteile und eine Behandlungseinheit zur Behandlung des oder der gewonnenen Blutbestandteile verfügt. Darüber hinaus betrifft die Erfindung ein Verfahren zur Überwachung der Flüssigkeitsströmung in einer derartigen extrakorporalen Blutbehandlungsvorrichtung.

Als therapeutische Apherese ist ein Blutreinigungsverfahren bekannt, bei dem Blut oder Blutplasma in einem extrakorporalen Kreislauf von pathogenen Substanzen befreit wird. Man unterscheidet zwischen der unselektiven Plasmapherese, bei der das Plasma vom Blut separiert und vollständig substituiert wird, und der selektiven Plasmapherese, bei der Plasma durch Filtration oder Adsorbtion von pathogenen Substanzen befreit wird. Krankheiten, die mit der therapeutischen Apherese behandelt werden, sind beispielsweise Autoimmunkrankheiten. Für die therapeutische Apherese können die bekannten extrakorporalen Blutbehandlungsvorrichtungen eingesetzt werden.

Die bekannten extrakorporalen Blutbehandlungsvorrichtungen für die Apherese verfügen über eine Einheit zur Gewinnung von Plasma, insbesondere einen Plasmafilter, und eine Behandlungseinheit zur Behandlung des gewonnenen Plasmas, insbesondere eine einen oder mehrere Filter oder Adsorber umfassende Reinigungseinheit.

Der Plasmafilter befindet sich im extrakorporalen Blutkreislauf der Blutbehandlungsvorrichtung. Von dem Patienten führt eine Blutzuführleitung zu dem Einlass des Plasmafilters und von dem Auslass des Plasmafilters führt eine Blutrückführleitung zum Patienten, sodass dem Patienten entnommenes Blut durch den Plasmafilter geleitet werden kann. Das in dem Plasmafilter gewonnene Plasma wird dann durch die Reinigungseinheit geleitet und das gereinigte Plasma wird wieder dem Blut des Patienten im extrakorporalen Blutkreislauf zugeführt.

Blut und Plasma werden mittels Pumpen in Schlauchleitungen gefördert, die vor Beginn der Blutbehandlung mit Flüssigkeit gefüllt und gespült werden. Die Spüllösung wird in einem Sammelbehältnis, insbesondere einem Beutel aufgefangen. Um Verkeimungen zu vermeiden, verbleibt der Sammelbeutel während der gesamten Behandlung, die drei bis fünf Stunden dauern kann, an dem Schlauchleitungssystem.

Bei den bekannten Blutbehandlungsvorrichtungen zur therapeutischen Apherese zweigt sich die von der Reinigungseinheit abgehende Leitung in einen zu der Blutrückführleitung führenden ersten Leitungsabschnitt und einen zu dem Sammelbehältnis führenden zweiten Leitungsabschnitt auf. Während des Behandlungsmodus strömt das gereinigte Plasma durch den ersten Leitungsabschnitt zu der Blutrückführleitung, während der zweite Leitungsabschnitt verschlossen ist. In dem Füll- und Spülmodus wird die Spüllösung hingegen durch den zweiten Leitungsabschnitt in den Sammelbeutel geleitet. Die Flüssigkeitsströmung wird mittels einer Wechselklemme umgeschaltet, in die beide Leitungsabschnitte der von der Reinigungseinheit abgehenden Leitung eingelegt sind.

Im Behandlungsmodus muss sichergestellt werden, dass das gereinigte Plasma nicht in den Sammelbeutel gelangt, während in dem Füll- und Spülmodus sichergestellt werden muss, dass die Spüllösung im Sammelbeutel gesammelt wird. Daher ist eine Überwachung der korrekten Funktion der Wechselklemme erforderlich.

Es sind Vorrichtungen zur therapeutischen Apherese bekannt, bei denen ein Druck- und Klemmentest durchgeführt wird, um festzustellen, ob die Schlauchleitung ordnungsgemäß in die Wechselklemme eingelegt ist. Es sind Wechselklemmen bekannt, die über einen Sicherheitsbügel verfügen, der sich beim Herausnehmen bzw. Herausrutschen der Schlauchleitung öffnet. Beim Öffnen des Sicherheitsbügels wird ein Alarm gegeben.

Aus der WO 91/15253 und US 7,686,778 B2 ist die Überwachung des Gewichts von Behältnissen zum Bereitstellen und Sammeln von Flüssigkeiten während einer extrakorporalen Blutbehandlung bekannt. Beispielsweise werden Waagen zur Bilanzierung von frischer und verbrauchter Dialysierflüssigkeit eingesetzt, die in einem Beutel bereitgestellt bzw. gesammelt wird. Auch ist die Überwachung des Füllstandes von Beuteln bekannt, um ein Leer- oder Überlaufen zu detektieren

Der Erfindung liegt die Aufgabe zugrunde, die Sicherheit einer extrakorporalen Blutbehandlungsvorrichtung weiter zu erhöhen.

Die Lösung dieser Aufgabe erfolgt erfindungsgemäß mit den Merkmalen der Patentansprüche 1 und 11. Bevorzugte Ausführungsformen der Erfindung sind Gegenstand der Unteransprüche.

Die US 2011/0040228 A1 beschreibt eine extrakorporale Blutbehandlungsvorrichtung, die über einen extrakorporalen Blutkreislauf mit einer Filtereinheit verfügt. Darüber hinaus weist die Blutbehandlungsvorrichtung eine Einrichtung zur Bereitstellung von Substituat und eine Einrichtung zum Abführen von Ultrafiltrat auf. Die Einrichtung zum Abführen von Ultrafiltrat verfügt über eine Ultrafiltrat-Recyclingeinrichtung, um das Ultrafiltrat wieder dem Blut des Patienten zuführen zu können. Die von der Ultrafiltrateinrichtung abgehende Schlauchleitung führt zu einer Umschalteinrichtung, mit der die Flüssigkeitsströmung zwischen einer zu dem extrakorporalen Blutkreislauf oder zu einem Sammelbehältnis zur Aufnahme von Ultrafiltrat führenden Leitung umgeschaltet werden kann.

Aus der DE 43 38 858 C1 ist eine Vorrichtung zur Elimination von Stoffen aus dem Blut eines Patienten bekannt, die über eine Filtereinheit zum Abtrennen von Plasma verfügt. Aus dem Plasma werden die zu eliminierenden Stoffe entfernt. Das kontinuierlich gewonnene Plasma wird in einem Speicher gesammelt und dem Patienten wieder zugeführt.

Die erfindungsgemäße Vorrichtung und das erfindungsgemäße Verfahren beruhen darauf, dass zur Überwachung der Flüssigkeitsströmung in der von der Blutbehandlungseinheit abgehenden Leitung die Veränderung des Gewichts des Sammelbehältnisses überwacht wird. Allein auf der Grundlage der Veränderung des Gewichts des Sammelbehältnisses kann auf einen fehlerhaften Zustand geschlossen werden. Die erfindungsgemäße

Überwachung der Flüssigkeitsströmung kann die bekannte Überwachung der Funktion der Wechselklemme ersetzen. Zur Erhöhung der Redundanz können aber auch beide Sicherheitseinrichtungen vorgesehen sein.

Die erfindungsgemäße Überwachungseinrichtung verfügt über Mittel zum Bestimmen des Gewichts des Sammelbehältnisses und eine Auswerteinheit, die derart ausgebildet ist, dass zur Überwachung der Flüssigkeitsströmung eine Veränderung des Gewichts des Sammelbehältnisses feststellbar ist. Bei den Mitteln zum Bestimmen des Gewichts kann es sich um eine konventionelle elektronische Waage handeln, auf die ein Sammelbeutel aufgelegt oder an der der Beutel aufgehängt werden kann.

Die erfindungsgemäße Überwachung der Flüssigkeitsströmung erlaubt eine kontinuierliche Überwachung der Flüssigkeitsströmung sowohl in einem Behandlungsmodus, in dem die Blutbehandlung durchgeführt wird, als auch in einem Füll- oder Spülmodus, in dem das Flüssigkeitssystem mit einer Flüssigkeit gefüllt bzw. gespült wird.

Die extrakorporale Blutbehandlungsvorrichtung sieht eine Steuereinheit zur Vorgabe des Behandlungsmodus oder des Füll- oder Spülmodus vor. In dem Behandlungsmodus werden eine oder mehrere Blutkomponenten, insbesondere Blutplasma, über den ersten Abschnitt der von der Blutbehandlungseinheit abgehenden Leitung zu der Blutrückführleitung gefördert, während im Füll- oder Spülmodus Flüssigkeit, insbesondere eine Spüllösung, über den zweiten Abschnitt der von der Blutbehandlungseinheit abgehenden Leitung in das Sammelbehältnis gefördert wird. Während des Behandlungsmodus oder des Füll- oder Spülmodus überwacht die Auswerteinheit die Veränderung des Gewichts des Sammelbehältnisses.

Für den Fall, dass die Steuereinheit den Behandlungsmodus vorgibt, erzeugt die Auswerteinheit ein erstes Steuersignal dann, wenn eine Gewichtszunahme vorzugsweise in einem vorgegebenen Zeitintervall festgestellt wird. Wenn eine Gewichtszunahme in dem vorgegebenen Zeitintervall festgestellt wird, kann darauf geschlossen werden, dass eine oder mehrere Blutkomponenten, insbesondere Blutplasma, während des Behandlungsmodus in das Sammelbehältnis gelangen. Dies kann nur bei einer fehlerhaften Flüssigkeitsströmung durch die von der Blutbehandlungseinheit abgehenden Leitung der Fall sein. Mit der Überwachung der Gewichtszunahme ist folglich eine kontinuierliche Überwachung des Plasmaflusses möglich, wobei sichergestellt wird, dass Plasma nicht unbeabsichtigt in die Umgebung abfließen kann, d.h. während der Blutbehandlung in den Sammelbeutel verworfen werden kann.

Für den Fall, dass die Steuereinheit den Füll- oder Spülmodus vorgibt, erzeugt die Auswerteinheit ein zweites Steuersignal dann, wenn nach Ablauf eines vorgegebenen Zeitintervalls nach dem Beginn des Füll- oder Spülmodus eine Gewichtszunahme vorzugsweise in einem vorgegebenen Zeitintervall nicht festgestellt wird. Wenn eine Gewichtszunahme in dem vorgegebenen Zeitintervall nicht festgestellt wird, kann darauf geschlossen werden, dass Spülflüssigkeit nicht in das Sammelbehältnis gelangt. Dann liegt wieder ein fehlerhafter Zustand vor. Dabei ist das vorgegebene Zeitintervall derart bemessen, dass sich die Schlauchleitungen des Flüssigkeitssystems vollständig mit Spülflüssigkeit füllen kann. Denn erst mit der vollständigen Befüllung der Schlauchleitungen des Flüssigkeitssystems mit Flüssigkeit kann eine Gewichtszunahme des Sammelbeutels 13 zu erwarten sein. Dies ist nach Ablauf des vorgegebenen Zeitintervalls aber der Fall.

Wenn die Steuereinheit das erste oder zweite Steuersignal erzeugt, kann ein akustischer und/oder optischer Alarm gegeben werden. Hierfür sieht die erfindungsgemäße Blutbehandlungsvorrichtung eine Alarmeinheit vor.

Von Vorteil ist, wenn die Alarmeinheit bei der Erzeugung des ersten Steuersignals ein erstes Alarmsignal und bei der Erzeugung des zweiten Steuersignals ein zweites Alarmsignal gibt, um zwischen den beiden fehlerhaften Zuständen während des Behandlungsmodus bzw. Füll- oder Spülmodus unterscheiden zu können.

Bei einer besonders bevorzugten Ausführungsform der Erfindung wird im Füll- oder Spülmodus wieder nach Ablauf eines vorgegebenen Zeitintervalls nach der vollständigen Befüllung der Schlauchleitungen des Flüssigkeitssystems der Betrag der Veränderung des Gewichts des Sammelbehältnisses in einem vorgegebenen Zeitintervall bestimmt und der Betrag der Veränderung des Gewichts in dem vorgegebenen Zeitintervall mit einem vorgegebenen Grenzwert verglichen, um nicht nur feststellen zu können, ob das Gewicht zunimmt, sondern auch feststellen zu können, um welchen Betrag das Gewicht zunimmt. Damit kann die Befüllung des Flüssigkeitssystems, insbesondere des Plasmakreises überwacht werden. Nach Beginn des Füll- oder Spülmodus kann auch überwacht werden, ob sich eine kontinuierliche Gewichtszunahme in einem vorgegebenen Zeitintervall einstellt. Erst wenn dies der Fall ist, kann darauf geschlossen werden, dass das Flüssigkeitssystem luftfrei ist.

Wenn die Gewichtszunahme unterhalb eines vorgegebenen Grenzwertes liegt, kann auf einen fehlerhaften Zustand geschlossen werden. Beispielsweise kann darauf geschlossen werden, dass die Schlauchleitung nicht ordnungsgemäß in der einzigen Wechselklemme zum Umschalten der Flüssigkeitsströmung oder in den beiden Schlauchklemmen sitzt.

Bei einer weiteren besonders bevorzugten Ausführungsform wird das Gewicht des Saminelbehältnisses zu Beginn des Füll- oder Spülmodus und nach Beendigung des Füll- oder Spülmodus festgestellt, um das geförderte Flüssigkeitsvolumen in dem Flüssigkeitssystems, insbesondere des Plasmakreises bestimmen zu können. Dies setzt aber wieder die vollständige Befüllung der Schlauchleitungen des Flüssigkeitssystems mit Flüssigkeit voraus.

Die Überwachung der Flüssigkeitsströmung auf der Grundlage des Gewichts des Sammelbehältnisses kann bei allen Blutbehandlungsvorrichtungen Verwendung finden, die über eine Einheit zur Gewinnung eines oder mehrerer Blutbestandteile und eine Behandlungseinheit zur Behandlung des oder der gewonnenen Blutbestandteile verfügt, wobei die behandelten Blutbestandteile dem Blut wieder zugeführt werden. Dabei ist es unerheblich, wie die Einheit zur Gewinnung der Blutbestandteile und die Behandlungseinheit beschaffen sind. Insbesondere ist die extrakorporale Blutbehandlungsvorrichtung eine Vorrichtung zur Apherese, wobei die Einheit zur Gewinnung eines oder mehrerer Blutbestandteile eine Einheit zur Gewinnung von Plasma, insbesondere ein Plasmafilter, und die Blutbehandlungseinheit eine Einheit zur Befreiung des Plasmas von pathogenen Bestandteilen, beispielsweise eine aus einem oder mehreren Filtern oder Adsorbern bestehende Reinigungseinheit, ist.

Die Mittel zum Umschalten der Flüssigkeitsströmung umfassen vorzugsweise eine erste Schlauchklemme zum Abklemmen des ersten Leitungsabschnitts der von der Blutbehandlungseinheit abgehenden Leitung, und eine zweite Schlauchklemme zum Abklemmen des zweiten Leitungsabschnitts der von der Blutbehandlungseinheit abgehenden Leitung. Beide Schlauchklemmen können aber auch eine Einheit bilden. Beispielsweise kann es sich um eine Wechselklemme handeln.

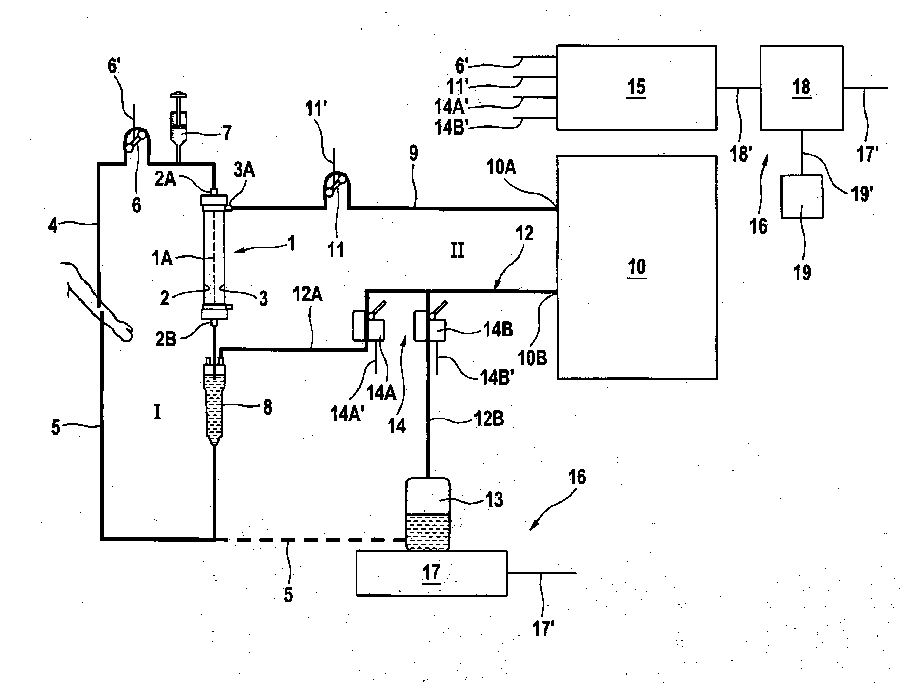

Im Folgenden wird ein Ausführungsbeispiel der Erfindung unter Bezugnahme auf die Zeichnung im Einzelnen erläutert.

Die Figur zeigt in einer stark vereinfachten schematischen Darstellung die wesentlichen Komponenten der extrakorporalen Blutbehandlungsvorrichtung, die über eine Einrichtung zur Überwachung der Flüssigkeitsströmung verfügt.

Die Einrichtung zur Überwachung der Flüssigkeitsströmung ist bei dem vorliegenden Ausführungsbeispiel Bestandteil der extrakorporalen Blutbehandlungsvorrichtung. Sie kann aber auch eine separate Baugruppe bilden. Bei der extrakorporalen Blutbehandlungsvorrichtung handelt es sich in dem vorliegenden Ausführungsbeispiel um eine Vorrichtung zur therapeutischen Apherese.

Die Blutbehandlungsvorrichtung umfasst einen extrakorporalen Blutkreislauf I, der eine Einheit 1 zur Gewinnung einer oder mehrerer Blutbestandteile aufweist. Bei dem vorliegenden Ausführungsbeispiel ist diese Einheit ein Plasmafilter 1, der durch eine semipermeable Membran 1A in eine erste Kammer 2 und eine zweite Kammer 3 unterteilt ist. Von dem Patienten führt eine Blutzuführleitung 4 zu dem Einlass 2A der ersten Kammer 2 des Plasmafilters 1. Von dem Auslass 2B der ersten Kammer 2 des Plasmafilters 1 führt eine Blutrückführleitung 5 wieder zu dem Patienten.

Zum Fördern des Bluts von dem Patienten in den Plasmafilter 1 dient eine Blutpumpe 6, die in die Blutzuführleitung 4 geschaltet ist. Dem Blut des Patienten kann über eine nur andeutungsweise dargestellte Heparinpumpe 7 Heparin stromauf des Plasmafilters 1 zugeführt werden. In die Blutrückführleitung 5 ist eine Tropfkammer 8 geschaltet.

Neben dem extrakorporalen Blutkreislauf I umfasst die Blutbehandlungsvorrichtung einen Sekundärkreislauf II, der nachfolgend als Plasmakreislauf II bezeichnet wird. Der Plasmakreislauf II umfasst eine Leitung 9, die von dem Auslass 3A der zweiten Kammer 3 des Plasmafilters 1 abgeht und zu dem Einlass 10A einer Blutbehandlungseinheit 10 führt. Die in der Figur nur andeutungsweise dargestellte Blutbehandlungseinheit 10 ist bei dem vorliegenden Ausführungsbeispiel eine Reinigungseinheit, die über einen oder mehrere Filter oder Adsorber verfügt, um das in dem Plasmafilter 1 aus dem Blut des Patienten gewonnene Plasma zu reinigen. Das Plasma wird mit einer Plasmapumpe 11, die in die Leitung 9 geschaltet ist, von dem Plasmafilter 1 zu der Blutbehandlungseinheit 10 gefördert.

Das gereinigte Plasma wird dem Blut des Patienten wieder zugeführt. Von dem Auslass 10B der Blutbehandlungseinheit 10 geht eine Leitung 12 ab, die sich in einen ersten Leitungsabschnitt 12A und einen zweiten Leitungsabschnitt 12B aufzweigt. Der erste Leitungsabschnitt 12A führt zu der Blutrückführleitung 5. Bei dem vorliegenden Ausführungsbeispiel ist der erste Leitungsabschnitt 12A an der in die Blutrückführleitung 5 geschalteten Tropfkammer 8 angeschlossen. Der zweite Leitungsabschnitt 12B der von der Blutbehandlungseinheit 10 abgehenden Leitung 12 führt zu einem Behältnis 13 zum Sammeln von Flüssigkeit, insbesondere ein Sammelbeutel.

Bei den Leitungen 4, 5 des extrakorporalen Blutkreislaufs I und den Leitungen 9, 12 des Plasmakreislaufs II handelt es sich um Schlauchleitungen eines oder mehrerer Schlauchleitungssets.

Der Sammelbeutel 13 dient zur Aufnahme einer Spülflüssigkeit. Für die Apherese bleibt der Beutel 13 fest mit dem Schlauchset verbunden, um das Risiko einer Verkeimung zu verringern.

Während der Apherese wird die Flüssigkeitsströmung in dem zu dem Sammelbeutel 13 führenden Schlauchleitungsabschnitt 12B der Schlauchleitung 12 unterbrochen, sodass das gereinigte Plasma nicht in den Sammelbeutel 13 gelangen kann. Während des Füll- oder Spülmodus hingegen wird Spülflüssigkeit durch den Leitungsabschnitt 12B der Leitung 12 in den Sammelbeutel 13 verworfen. Darüber hinaus kann im Füll- und Spülmodus die Blutrückführleitung 5 mit dem Sammelbeutel 13 verbunden werden. Der Anschluss der Blutrückführleitung 5 an den Sammelbeutel 13 wird in der Fig. durch eine gestrichelte Linie angedeutet. Mit dem Anschluss der Blutrückführleitung 5 an den Sammelbeutel 13 wird der Sammelbeutel im Füll- und Spülmodus sowohl über den zweiten Leitungsabschnitt 12B der von der Blutbehandlungseinheit 10 abgehenden Leitung 12 als auch über die Blutrückführleitung 5 befüllt.

Die Mittel zum Umschalten der Flüssigkeitsströmung 14 sind in der Figur nur schematisch dargestellt. Bei dem vorliegenden Ausführungsbeispiel handelt es sich um zwei Schlauchklemmen 14A, 14B, die wechselweise betätigt werden. Die Schlauchleitungsabschnitte 12A und 12B der Schlauchleitung 12 müssen vor dem Füll- oder Spülmodus bzw. vor der Blutbehandlung in die Schlauchklemmen 14A, 14B eingelegt werden, um die jeweiligen Leitungsabschnitte 12A, 12B abklemmen zu können. Darüber hinaus verfügt die Blutbehandlungsvorrichtung über eine zentrale Steuereinheit 15, die über Steuerleitungen 6', 11' mit der Blutpumpe 6 und der Plasmapumpe 11 verbunden ist.

Die Einrichtung 16 zur Überwachung der Flüssigkeitsströmung weist Mittel 17 zum Wiegen des Sammelbeutels 13, eine Auswerteinheit 18 und eine Alarmeinheit 19 auf. Die Auswerteinheit 18 ist über eine Datenleitung 18' mit der zentralen Steuereinheit 15 der Blutbehandlungsvorrichtung verbunden, während die Alarmeinheit 19 mit einer Datenleitung 19' mit der Auswerteinheit 18 verbunden ist. Die Auswerteinheit 18 kann aber auch Bestandteil der zentralen Steuereinheit 15 der Blutbehandlungsvorrichtung sein. Die Mittel 17 zum Wiegen des Sammelbeutels 13 sind über eine Datenleitung 17' mit der Auswerteinheit 18 verbunden. Die Schlauchklemmen 14A, 14B der Mittel 14 zum Umschalten der Flüssigkeitsströmung können von Hand betätigbare Schlauchklemmen sein. Bei dem vorliegenden Ausführungsbeispiel werden die Schlauchklemmen über Steuerleitungen 14A', 14B' von der zentralen Steuereinheit 15 wechselweise betätigt.

Die zentrale Steuereinheit 15 kann einen Behandlungsmodus und einen Füll- und Spülmodus vorgeben. Im Behandlungsmodus öffnet die Steuereinheit 15 die erste Schlauchklemme 14A, die zum Abklemmen des ersten Abschnitts 12A der Leitung 12 dient, und schließt die zweite Schlauchklemme 14B, die zum Abklemmen des zweiten Abschnitts 12B der Leitung 12 dient, sodass Plasma nicht in den Sammelbeutel 13 gelangen kann. Im Füll- und Spülmodus hingegen öffnet die Steuereinheit 15 die zweite Schlauchklemme 14B, sodass Spülflüssigkeit in den Sammelbeutel 13 verworfen werden kann.

Neben den oben beschrieben Komponenten kann die Blutbehandlungsvorrichtung noch über weitere Komponenten, beispielsweise einen Antikoagulanz-Beutel und eine Antikoagulanz-Pumpe oder weitere Schlauchklemmen, insbesondere eine venöse Klemme, verfügen, die aber in der Figur nicht dargestellt sind.

Nachfolgend wird die Funktionsweise der Überwachungseinrichtung im Einzelnen beschrieben.

Es wird angenommen, dass die zentrale Steuereinheit 15 den Behandlungsmodus vorgibt. Im Behandlungsmodus muss sichergestellt sein, dass die zweite Schlauchklemme 14B geschlossen ist. Darüber hinaus muss sichergestellt sein, dass der zweite Abschnitt 12B der Leitung 12 in die zweite Schlauchklemme 14B eingelegt ist. Ansonsten könnte die Schlauchklemme den Leitungsabschnitt nicht abklemmen, sodass Plasma in den Sammelbeutel 13 gelangt.

Die Auswerteinheit 18 empfängt während der Blutbehandlung laufend die Signale der Mittel 17 zum Wiegen des Gewichts des Sammelbeutels, um feststellen zu können, ob das Gewicht zunimmt oder konstant bleibt. Wenn die Auswerteinheit 18 eine Gewichtszunahme detektiert, wird ein erstes Steuersignal erzeugt, dass die Alarmeinheit 19 empfängt. Daraufhin gibt die Alarmeinheit einen ersten akustischen oder optischen Alarm, der darauf hinweist, das Plasma in die Umgebung gelangt. Das erste Steuersignal empfängt auch die zentrale Steuereinheit 15, die einen Eingriff in die Maschinensteuerung vornehmen kann. Beispielsweise kann die Steuereinheit 15 die Blutbehandlung unterbrechen. Die Auswerteinheit 18 überprüft also, ob das Gewicht des Sammelbehältnisses 13 während der Blutbehandlung zunimmt. Solange das Gewicht nicht zunimmt, ist ein ordnungsgemäßer Betrieb sichergestellt.

In dem Füll- oder Spülmodus muss sichergestellt sein, dass die zweite Schlauchklemme 14B geöffnet ist, sodass Spülflüssigkeit in den Plasmabeutel 13 zur vollständigen luftfreien Befüllung des gesamten Plasmakreislaufs II verworfen wird. Wenn die Steuereinheit 15 den Füll- und Spülmodus vorgibt, überwacht die Auswerteinheit 18 wieder das Gewicht des Sammelbehältnisses 13. Die Auswerteinheit 18 erzeugt ein zweites Steuersignal, wenn eine Gewichtszunahme nicht nach Ablauf eines vorgegebenen Zeitintervalls nach dem Start des Füll- und Spülmodus festgestellt wird. In diesem Fall ist die zweite Schlauchklemme 14B geschlossen. Das vorgegebene Zeitintervall ist derart bemessen, dass sich das Flüssigkeitssystem vollständig mit Spülflüssigkeit füllen kann. Denn vor dem Füll- oder Spülmodus oder zu Beginn der Füll- oder Spülphase werden in der Praxis die Schlauchleitungen des Flüssigkeitssystems nicht vollständig mit Flüssigkeit gefüllt sein, da sich zumindest in Teilen des Flüssigkeitssystems Luft befindet. Erst wenn die Schlauchleitungen des Flüssigkeitssystems vollständig mit Flüssigkeit gefüllt sind, kann eine Gewichtszunahme des Sammelbeutels 13 zu erwarten sein. Dies ist nach Ablauf des vorgegebenen Zeitintervalls aber der Fall.

Wenn das zweite Steuersignal erzeugt wird, gibt die Alarmeinheit 19 ein zweites akustisches und/oder optisches Alarmsignal, das auf diesen fehlerhaften Zustand hinweist. Auch die Steuereinheit 15 empfängt das zweite Steuersignal, um einen Eingriff in die Maschinensteuerung vornehmen zu können. Beispielsweise kann die Steuereinheit den Füll- oder Spülmodus unterbrechen.

Die Auswerteinheit 18 überwacht bei dem vorliegenden Ausführungsbeispiel nicht nur, ob eine Gewichtszunahme vorlegt, sondern überwacht auch den Betrag der Gewichtszunahme pro Zeiteinheit. Sie bestimmt den Betrag der Veränderung des Gewichts des Sammelbehältnisses 13 in einem vorgegebenen Zeitintervall und vergleicht den Betrag der Veränderung des Gewichts in dem vorgegebenen Zeitintervall mit einem vorgegebenen Wert. Wenn der Betrag der Veränderung des Gewichts in dem vorgegebenen Zeitintervall kleiner als ein vorgegebener Grenzwert ist, wird ein drittes Steuersignal erzeugt, dass einen dritten Alarm oder einen weiteren Eingriff in die Maschinensteuerung auslösen kann. Dadurch ist es möglich, den Fluss der Spüllösung in das Sammelbehältnis 13 zu überwachen. Wenn bei einem fehlerhaften Zustand der Flüssigkeitsfluss unter einen Sollwert sinkt, nimmt der ansonsten kontinuierliche Zuwachs des spezifischen Gewichts des Sammelbehältnisses 13 ab, sodass der Betrag der Veränderung des Gewichts unter den vorgegebenen Wert fällt. Auch bei der Überwachung des Betrags der Gewichtszunahme pro Zeiteinheit erfolgt die Erzeugung des dritten Steuersignal erst nach Ablauf eines vorgegebenen Zeitintervalls nach dem Start des Füll- oder Spülmodus, das derart bemessen ist, dass in diesem Zeitintervall eine vollständige Befüllung des Flüssigkeitssystem zu erwarten ist.

Neben der Überwachung des Gewichtszuwachses des Sammelbehältnisses in einem bestimmten vorgegebenen Zeitintervall des Füll- oder Spülmodus kann die Auswerteinheit 18 grundsätzlich auch eine Überwachung der Gewichtszunahme während der gesamten Füll- oder Spülphase vorsehen, was aber voraussetzt, dass das Flüssigkeitssystem zuvor vollständig mit Flüssigkeit befüllt ist, bevor die Überwachung beginnt. Hierzu bestimmt die Auswerteinheit das Gewicht des Sammelbehältnisses 13 zu Beginn des Füll- oder Spülmodus zu einem Zeitpunkt, zu dem das Flüssigkeitssystem vollständig mit Flüssigkeit befüllt ist, und nach Beendigung des Füll- und Spülmodus. Aus der Differenz des Gewichts des Sammelbehältnisses 13 vor Beginn und nach Beendigung des Füll- oder Spülmodus ermittelt die Auswerteinheit 18 dann das geförderte Flüssigkeitsvolumen in dem Plasmakreis II. Für die Berechnung des geförderten Flüssigkeitsvolumens wird das spezifische Gewicht der Spülflüssigkeit in einem Speicher der Auswerteinheit 18 abgelegt. Wenn das gemessene Flüssigkeitsvolumen von dem tatsächlichen Flüssigkeitsvolumen des Schlauchsystems abweicht, kann die Auswerteinheit 18 wieder ein Steuersignal erzeugen, das auf einen fehlerhaften Zustand hinweist und einen Alarm auslösen kann.

## Patentansprüche

1. Vorrichtung zur extrakorporalen Blutbehandlung mit
einer zu einer Einheit (1) zur Gewinnung eines oder mehrerer Blutbestandteile führenden Blutzuführleitung (4),
einer von der Einheit (1) zur Gewinnung eines oder mehrerer Blutbestandteile abgehenden Blutrückführleitung (5),
einer zu einer Behandlungseinheit (10) zur Behandlung des oder der gewonnenen Blutbestandteile führenden Leitung (9),
einer von der Behandlungseinheit (10) abgehenden Leitung (12), die einen zu der Blutrückführleitung (5) führenden ersten Leitungsabschnitt (12A) und einen zu einem Sammelbehältnis (13) führenden zweiten Leitungsabschnitt (12B) aufweist und
Mitteln (14) zum Umschalten der Flüssigkeitsströmung zwischen einer ersten Richtung, in der Flüssigkeit durch den ersten Leitungsabschnitt (12A) der von der Behandlungseinheit abgehenden Leitung (12) strömt, und einer zweiten Richtung, in der Flüssigkeit durch den zweiten Leitungsabschnitt (12B) der von der Behandlungseinheit abgehenden Leitung (12) strömt,
**dadurch gekennzeichnet, dass**
eine Einrichtung (16) zur Überwachung der Flüssigkeitsströmung durch den ersten oder zweiten Leitungsabschnitt (12A, 12B) der von der Behandlungseinheit abgehenden Leitung (12) vorgesehen ist, wobei
die Überwachungseinrichtung (16) Mittel (17) zum Bestimmen des Gewichts des Sammelbehältnisses (13) und eine Auswerteinheit (18) aufweist, die derart ausgebildet ist, dass zur Überwachung der Flüssigkeitsströmung eine Veränderung des Gewichts des Sammelbehälnisses feststellbar ist.

2. Vorrichtung nach Anspruch 1, **dadurch gekennzeichnet, dass** die extrakorporale Blutbehandlungsvorrichtung eine Steuereinheit (15) zur Vorgabe eines Behandlungsmodus aufweist, in dem eine oder mehrere Blutkomponenten über den ersten Leitungsabschnitt (12A) der von der Blutbehandlungseinheit abgehenden Leitung (12) zu der Blutrückführleitung (5) gefördert werden, wobei die Auswerteinheit (18) derart ausgebildet ist, dass in dem Behandlungsmodus eine Gewichtszunahme des Sammelbehältnisses feststellbar ist, wobei ein erstes Steuersignal erzeugt wird, wenn eine Gewichtszunahme festgestellt wird.

3. Vorrichtung nach Anspruch 1, **dadurch gekennzeichnet, dass** die extrakorporale Blutbehandlungsvorrichtung eine Steuereinheit (15) zur Vorgabe eines Füll- oder Spülmodus aufweist, in dem Flüssigkeit über den zweiten Leitungsabschnitt (12B) der von der Blutbehandlungseinheit abgehenden Leitung (12) in das Sammelbehältnis (13) gefördert wird, wobei die Auswerteinheit (18) derart ausgebildet ist, dass in dem Füll- oder Spülmodus nach Ablauf eines vorgegebenen Zeitintervalls nach dem Beginn des Füll- oder Spülmodus eine Gewichtszunahme des Sammelbehältnisses feststellbar ist, wobei ein zweites Steuersignal erzeugt wird, wenn eine Gewichtszunahme nach Ablauf des vorgegebenen Zeitintervalls nicht festgestellt wird.

4. Vorrichtung nach Anspruch 3, **dadurch gekennzeichnet, dass** die Auswerteinheit (18) derart ausgebildet ist, dass nach Ablauf eines vorgegebenen Zeitintervalls nach dem Beginn des Füll- oder Spülmodus der Betrag der Veränderung des Gewichts des Sammelbehältnisses (13) in einem vorgegebenen Zeitintervall bestimmt und der Betrag der Veränderung des Gewichts in dem vorgegebenen Zeitintervall mit einem vorgegebenen Wert verglichen wird, wobei das zweite Steuersignal erzeugt wird, wenn der Betrag der Veränderung des Gewichts in dem vorgegebenen Zeitintervall kleiner als der vorgegebene Wert ist.

5. Vorrichtung nach Anspruch 3 oder 4, **dadurch gekennzeichnet, dass** die Auswerteinheit (18) derart ausgebildet ist, dass das Gewicht des Sammelbehälnisses (13) zu Beginn des Füll- oder Spülmodus und nach Beendigung des Füll- oder Spülmodus festgestellt wird.

6. Vorrichtung nach Anspruch 5, **dadurch gekennzeichnet, dass** die Auswerteinheit (18) derart ausgebildet ist, dass aus der Differenz des Gewichts des Sammelbehältnisses (13) zu Beginn des Füll- oder Spülmodus und nach Beendigung des Füll- oder Spülmodus das geförderte Flüssigkeitsvolumen in dem Flüssigkeitssystems ermittelt wird.

7. Vorrichtung nach einem der Ansprüche 1 bis 6, **dadurch gekennzeichnet, dass** eine mit der Auswerteinheit (18) zusammenwirkende Alarmeinheit (19) vorgesehen ist, die einen akustischen und/oder optischen Alarm erzeugt, wenn die Auswerteinheit ein Steuersignal erzeugt.

8. Vorrichtung nach einem der Ansprüche 1 bis 7, **dadurch gekennzeichnet, dass** die extrakorporale Blutbehandlungsvorrichtung eine Vorrichtung zur Apherese ist, wobei die Einheit (1) zur Gewinnung eines oder mehrerer Blutbestandteile eine Einheit zur Gewinnung von Plasma, insbesondere ein Plasmafilter ist.

9. Vorrichtung nach Anspruch 8, **dadurch gekennzeichnet, dass** die Blutbehandlungseinheit (10) eine Einheit zur Befreiung des Plasmas von pathogenen Bestandteilen ist.

10. Vorrichtung nach einem der Ansprüche 1 bis 9, **dadurch gekennzeichnet, dass** die Mittel (14) zum Umschalten der Flüssigkeitsströmung eine erste Schlauchklemme (14A) zum Abklemmen des ersten Leitungsabschnitts (12A) der von der Behandlungseinheit abgehenden Leitung (12), und eine zweite Schlauchklemme (14B) zum Abklemmen des zweiten Leitungsabschnitts (12B) der von der Behandlungseinheit abgehenden Leitung (12) aufweisen.

11. Verfahren zur Überwachung der Flüssigkeitsströmung in einer Vorrichtung zur extrakorporalen Blutbehandlung, wobei die Vorrichtung zur extrakorporalen Blutbehandlung aufweist: ,
eine zu einer Einheit zur Gewinnung eines oder mehrerer Blutbestandteile führenden Blutzuführleitung,
eine von der Einheit zur Gewinnung eines oder mehrerer Blutbestandteile abgehenden Blutrückführleitung,
eine zu einer Behandlungseinheit zur Behandlung des oder der gewonnenen Blutbestandteile führenden Leitung,
eine von der Behandlungseinheit abgehenden Leitung, die einen zu der Blutrückführleitung führenden ersten Leitungsabschnitt und einen zu einem Sammelbehältnis führenden zweiten Leitungsabschnitt aufweist,
Mittel zum Umschalten der Flüssigkeitsströmung zwischen einer ersten Richtung, in der Flüssigkeit durch den ersten Leitungsabschnitt der von der Behandlungseinheit abgehenden Leitung strömt, und einer zweiten Richtung, in der Flüssigkeit durch den zweiten Leitungsabschnitt der von der Behandlungseinheit abgehenden Leitung strömt,
**dadurch gekennzeichnet, dass**
zur Überwachung der Flüssigkeitsströmung durch den ersten oder zweiten Leitungsabschnitt der von der Behandlungseinheit abgehenden Leitung die Veränderung des Gewichts des Sammelbehälnisses überwacht wird.

12. Verfahren nach Anspruch 11, **dadurch gekennzeichnet, dass** bei der Vorgabe eines Behandlungsmodus für die extrakorporale Blutbehandlungsvorrichtung, in dem eine oder mehrere Blutkomponenten über den ersten Leitungsabschnitt der von der Blutbehandlungseinheit abgehenden Leitung zu der Blutrückführleitung gefördert werden, die Gewichtszunahme des Sammelbehältnisses in dem Behandlungsmodus überwacht wird, wobei auf einen fehlerhaften Zustand geschlossen wird, wenn eine Gewichtszunahme festgestellt wird.

13. Verfahren nach Anspruch 11, **dadurch gekennzeichnet, dass** bei der Vorgabe eines Füll- oder Spülmodus, in dem Flüssigkeit über den zweiten Leitungsabschnitt der von der Blutbehandlungseinheit abgehenden Leitung in das Sammelbehältnis gefördert wird, nach Ablauf eines vorgegebenen Zeitintervalls nach dem Beginn des Füll- oder Spülmodus eine Gewichtszunahme des Sammelbehältnisses in dem Füll- oder Spülmodus überwacht wird, wobei auf einen fehlerhaften Zustand geschlossen wird, wenn eine Gewichtszunahme nach Ablauf des vorgegebenen Zeitintervalls nicht festgestellt wird.

14. Verfahren nach Anspruch 13, **dadurch gekennzeichnet, dass** nach Ablauf eines vorgegebenen Zeitintervalls nach dem Beginn des Füll- oder Spülmodus der Betrag der Veränderung des Gewichts des Sammelbehältnisses in einem vorgegebenen Zeitintervall bestimmt und der Betrag der Veränderung des Gewichts in dem vorgegebenen Zeitintervall mit einem vorgegebenen Wert verglichen wird, wobei auf einen fehlerhaften Zustand geschlossen wird, wenn der Betrag der Veränderung des Gewichts in dem vorgegebenen Zeitintervall kleiner als der vorgegebene Wert ist.

15. Verfahren nach Anspruch 13 oder 14, **dadurch gekennzeichnet, dass** das Gewicht des Sammelbehälnisses zu Beginn des Füll- oder Spülmodus und nach Beendigung des Füll- oder Spülmodus gemessen wird.

16. Verfahren nach Anspruch 15, **dadurch gekennzeichnet, dass** aus der Differenz des Gewichts des Sammelbehältnisses zu Beginn des Füll- oder Spülmodus und nach Beendigung des Füll- oder Spülmodus das geförderte Flüssigkeitsvolumen in dem Flüssigkeitssystems ermittelt wird.

17. Verfahren nach einem der Ansprüche 11 bis 16, **dadurch gekennzeichnet, dass** nach Feststellung eines fehlerhaften Zustandes ein akustischer und/oder optischer Alarm gegeben wird.

18. Verfahren nach einem der Ansprüche 11 bis 17, **dadurch gekennzeichnet, dass** die extrakorporale Blutbehandlungsvorrichtung eine Vorrichtung zur Apherese ist, wobei die Einheit zur Gewinnung eines oder mehrerer Blutbestandteile eine Einheit zur Gewinnung von Plasma, insbesondere ein Plasmafilter, und die Einheit zur Blutbehandlung eine Einheit zur Befreiung des Plasmas von pathogenen Bestandteilen ist.

## Claims

1. Device for extracorporeal blood treatment, comprising
a blood supply line (4) leading to a unit (1) for obtaining one or more blood constituents,
a blood return line (5) leading away from the unit (1) for obtaining one or more blood constituents,
a line (9) leading to a treatment unit (10) for treating the obtained blood constituent(s),
a line (12) which leads away from the treatment unit (10) and comprises a first line portion (12A) leading to the blood return line (5) and a second line portion (12B) leading to a collection container (13), and
means (14) for switching the fluid flow between a first direction in which fluid flows through the first line portion (12A) of the line (12) leading away from the treatment unit, and a second direction in which fluid flows through the second line portion (12B) of the line (12) leading away from the treatment unit,
**characterised in that**
an apparatus (16) is provided for monitoring the fluid flow through the first or second line portion (12A, 12B) of the line (12) leading away from the treatment unit, the monitoring apparatus (16) comprising means (17) for determining the weight of the collection container (13) and an evaluation unit (18) which is designed in such a way that, in order to monitor the fluid flow, a change in the weight of the collection container can be ascertained.

2. Device according to claim 1, **characterised in that** the extracorporeal blood treatment device comprises a control unit (15) for selecting a treatment mode, in which one or more blood components are conveyed to the blood return line (5) via the first line portion (12A) of the line (12) leading away from the blood treatment unit, the evaluation unit (18) being designed in such a way that, in the treatment mode, an increase in the weight of the collection container can be ascertained, a first control signal being generated if an increase in weight is ascertained.

3. Device according to claim 1, **characterised in that** the extracorporeal blood treatment device comprises a control unit (15) for selecting a filling or rinsing mode, in which fluid is conveyed to the collection container (13) via the second line portion (12B) of the line (12) leading away from the blood treatment unit, the evaluation unit (18) being designed in such a way that, in the filling or rinsing mode, an increase in the weight of the collection container can be ascertained after the end of a preset time interval after the filling or rinsing mode has started, a second control signal being generated if an increase in weight is not ascertained after the end of the preset time interval.

4. Device according to claim 3, **characterised in that** the evaluation unit (18) is designed in such a way that the extent to which the weight of the collection container (13) changes during a preset time interval is determined after the end of a preset time interval once the filling or rinsing mode has started, and the extent to which the weight changes during the preset time interval is compared with a preset value, the second control signal being generated if the extent to which the weight changes during the preset time interval is less than the preset value.

5. Device according to either claim 3 or claim 4, **characterised in that** the evaluation unit (18) is designed in such a way that the weight of the collection container (13) is ascertained at the start of the filling or rinsing mode and after completion of the filling or rinsing mode.

6. Device according to claim 5, **characterised in that** the evaluation unit (18) is designed in such a way that the conveyed volume of fluid in the fluid system is determined from the difference in weight of the collection container (13) at the start of the filling or rinsing mode and after completion of the filling or rinsing mode.

7. Device according to any of claims 1 to 6, **characterised in that** an alarm unit (19) is provided which interacts with the evaluation unit (18) and generates an acoustic and/or visual alarm when the evaluation unit generates a control signal.

8. Device according to any of claims 1 to 7, **characterised in that** the extracorporeal blood treatment device is an apheresis device, the unit (1) for obtaining one or more blood constituents being a unit for obtaining plasma, in particular being a plasma filter.

9. Device according to claim 8, **characterised in that** the blood treatment unit (10) is a unit for freeing the plasma from pathogenic constituents.

10. Device according to any of claims 1 to 9, **characterised in that** the means (14) for switching the fluid flow comprise a first hose clamp (14A) for pinching off the first line portion (12A) of the line (12) leading away from the treatment unit and a second hose clamp (14B) for pinching off the second line portion (12B) of the line (12) leading away from the treatment unit.

11. Method for monitoring the fluid flow in a device for extracorporeal blood treatment, the device for extracorporeal blood treatment comprising:
a blood supply line leading to a unit for obtaining one or more blood constituents,
a blood return line leading away from the unit for obtaining one or more blood constituents,
a line leading to a treatment unit for treating the obtained blood constituent(s),
a line which leads away from the treatment unit and comprises a first line portion leading to the blood return line and a second line portion leading to a collection container,
means for switching the fluid flow between a first direction in which fluid flows through the first line portion of the line leading away from the treatment unit, and a second direction in which fluid flows through the second line portion of the line leading away from the treatment unit,
**characterised in that**
the change in the weight of the collection container is monitored in order to monitor the fluid flow through the first or second line portion of the line leading away from the treatment unit.

12. Method according to claim 11, **characterised in that**, when selecting a treatment mode for the extracorporeal blood treatment device, in which one or more blood components are conveyed to the blood return line via the first line portion of the line leading away from the blood treatment unit, the increase in the weight of the collection container, when in the treatment mode, is monitored, a defective state being concluded if an increase in weight is ascertained.

13. Method according to claim 11, **characterised in that**, when selecting a filling or rinsing mode, in which fluid is conveyed to the collection container via the second line portion of the line leading away from the blood treatment unit, an increase in the weight of the collection container, when in the filling or rinsing mode, is monitored after the end of a preset time interval once the filling or rinsing mode has started, a defective state being concluded if an increase in weight is not ascertained after the end of the preset time interval.

14. Method according to claim 13, **characterised in that**, after the end of a preset time interval once the filling or rinsing mode has started, the extent to which the weight of the collection container changes during a preset time interval is determined and the extent to which the weight changes during the preset time interval is compared with a preset value, a defective state being concluded if the extent to which the weight changes during the preset time interval is less than the preset value.

15. Method according to either claim 13 or claim 14, **characterised in that** the weight of the collection container is measured at the start of the filling or rinsing mode and after completion of the filling or rinsing mode.

16. Method according to claim 15, **characterised in that** the conveyed volume of fluid in the fluid system is determined from the difference in weight of the collection container at the start of the filling or rinsing mode and after completion of the filling or rinsing mode.

17. Method according to any of claims 11 to 16, **characterised in that** an acoustic and/or visual alarm is emitted after a defective state has been ascertained.

18. Method according to any of claims 11 to 17, **characterised in that** the extracorporeal blood treatment device is an apheresis device, the unit for obtaining one or more blood constituents being a unit for obtaining plasma, in particular being a plasma filter, and the unit for blood treatment being a unit for freeing the plasma from pathogenic constituents.

## Revendications

1. Dispositif pour le traitement extracorporel du sang comprenant
une conduite d'amenée (4) du sang conduisant à une unité (1) destinée à l'obtention d'un ou plusieurs constituants du sang,
une conduite de retour (5) du sang partant de l'unité (1) destinée à l'obtention d'un ou plusieurs constituants du sang,
une conduite (9) conduisant à une unité de traite-ment (10) pour traiter le ou les constituants du sang obtenus,
un conduite (12) partant de l'unité de traitement (10), présentant un premier tronçon de conduite (12A) conduisant vers la conduite de retour (5) et un deuxième tronçon de conduite (12B) conduisant vers un collecteur (13) et
des moyens (14) destinés à inverser le sens d'écoulement du liquide entre un premier sens, dans lequel du liquide s'écoule à travers le premier tronçon de conduite (12A) de la conduite (12) partant de l'unité de traitement, et un deuxième sens dans lequel du liquide s'écoule à travers le deuxième tronçon de conduite (12B) de la conduite (12) partant de l'unité de traitement,
**caractérisé en ce qu'**un dispositif (16) est prévu pour surveiller l'écoulement du liquide à travers le premier ou le deuxième tronçon de conduite (12A, 12B) de la conduite (12) partant de l'unité de traitement,
le dispositif de surveillance (16) présentant des moyens (17) pour déterminer le poids du collecteur (13) et une unité d'analyse (18) conçue de manière à ce qu'une modification du poids du collecteur peut être constatée pour surveiller l'écoulement du liquide.

2. Dispositif selon la revendication 1, **caractérisé en ce que** le dispositif de traitement extracorporel du sang présente une unité de commande (15) destinée à prédéfinir un mode de traitement dans lequel un ou plusieurs constituants du sang sont acheminés vers la conduite de retour (5) du sang par un premier tronçon de conduite (12A) de la conduite (12) partant de l'unité de traitement du sang, l'unité d'analyse (18) étant conçue de manière à pouvoir constater, dans le mode de traitement, une augmentation du poids du collecteur, un premier signal de commande étant généré lorsqu'une augmen-tation du poids est constatée.

3. Dispositif selon la revendication 1, **caractérisé en ce que** le dispositif de traitement extracorporel du sang présente une unité de commande (15) conçue pour prédéfinir un mode de remplissage ou de lavage dans lequel du liquide est acheminé vers le collecteur (13) par le deuxième tronçon de conduite (12B) de la conduite (12) partant de l'unité de traitement du sang, l'unité d'analyse (18) étant conçue de manière à pouvoir constater une augmenta-tion du poids du collecteur, en mode de remplissage ou de lavage, à l'issue d'un laps de temps pré-défini après le début du mode de remplissage ou de lavage, un deuxième signal de commande étant généré lorsqu'aucune augmentation de poids n'est constatée à l'issue du laps de temps prédéfini.

4. Dispositif selon la revendication 3, **caractérisé en ce que** l'unité d'analyse (18) est conçue de manière à ce que, à l'issue d'un laps de temps prédéfini après le début du mode de remplissage ou de lavage, la valeur de la modification du poids du collecteur (13) est déterminée dans un laps de temps prédéfini et la valeur de la modification du poids dans le laps de temps prédéfini est comparée à une valeur prédéfinie, le deuxième signal de commande étant généré lorsque la valeur de la modification du poids dans le laps de temps prédéfini est infé-rieure à la valeur prédéfinie.

5. Dispositif selon la revendication 3 ou la revendi-ation 4, **caractérisé en ce que** l'unité d'analyse (18) est conçue de manière à constater le poids du collecteur (13) au début du mode de remplissage ou de lavage et à l'issue du mode de remplissage ou de lavage.

6. Dispositif selon la revendication 5, **caractérisé en ce que** l'unité d'analyse (18) est conçue de manière à calculer le volume de liquide acheminé dans le système de liquide à partir de la différence entre le poids du collecteur (13) au début du mode de remplissage ou de lavage et à l'issue du mode de remplissage ou de lavage.

7. Dispositif selon l'une quelconque des revendica-tions 1 à 6, **caractérisé en ce qu'**une unité d'alarme (19), coopérant avec l'unité d'analyse (18), génère une alarme acoustique et/ou optique lorsque l'unité d'analyse génère un signal de commande.

8. Dispositif selon l'une quelconque des revendica-tions 1 à 7, **caractérisé en ce que** le dispositif de traitement extracorporel du sang est un dispositif d'aphérèse, l'unité (1) destinée à l'obtention d'un ou plusieurs constituants du sang étant une unité destinée à l'obtention de plasma, en particulier un filtre à plasma.

9. Dispositif selon la revendication 8, **caractérisé en ce que** l'unité de traitement du sang (10) est une unité destinée à enlever les constituants patho-gènes du plasma.

10. Dispositif selon l'une quelconque des revendica-tions 1 à 9, **caractérisé en ce que** les moyens (14) destinés à inverser le sens d'écoulement du liquide présentent un premier moyen de serrage (14A) pour semer le premier tronçon de conduite (12A) de la conduite (12) partant de l'unité de traitement, et un deuxième moyen de serrage (14B) pour semer le deuxième tronçon de conduite (12B) de la conduite (12) partant de l'unité de traitement.

11. Procédé de surveillance de l'écoulement du liquide dans un dispositif pour le traitement extracorporel du sang comprenant
une conduite d'amenée du sang conduisant à une unité destinée à l'obtention d'un ou plusieurs constituants du sang,
une conduite de retour du sang partant de l'unité destinée à l'obtention d'un ou plusieurs consti-tuants du sang,
une conduite conduisant à une unité de traitement pour traiter le ou les constituants du sang obtenus,
une conduite partant de l'unité de traitement, présentant un premier tronçon de conduite conduisant vers la conduite de retour et un deuxième tronçon de conduite conduisant vers un collecteur et
des moyens destinés à inverser le sens d'écoulement du liquide entre un premier sens, dans lequel du liquide s'écoule à travers le premier tronçon de conduite de la conduite partant de l'unité de traitement, et un deuxième sens dans lequel du liquide s'écoule à travers le deuxième tronçon de conduite de la conduite partant de l'unité de traitement,
**caractérisée en ce que**
la modification du poids du collecteur est surveillée afin de surveiller l'écoulement du liquide à travers le premier ou le deuxième tronçon de conduite de la conduite partant de l'unité de traitement.

12. Procédé selon la revendication 11, **caractérisé en ce que**, lors de la prédéfinition d'un mode de traitement pour le dispositif de traitement extracorporel du sang, dans lequel un ou plusieurs constituants du sang sont acheminés par le premier tronçon de conduite de la conduite partant de l'unité de traitement du sang vers la conduite de retour du sang, l'augmentation du poids du collec-teur est surveillée en mode de traitement, un état défectueux étant décelé lorsqu'une augmentation du poids est constatée.

13. Procédé selon la revendication 11, **caractérisé en ce que**, lors de la prédéfinition d'un mode de rem-plissage ou de lavage, dans lequel du liquide est acheminé vers le collecteur par le deuxième tronçon de conduite de la conduite partant de l'unité de traitement du sang, une augmentation du poids du collecteur est surveillée en mode de remplissage ou de lavage à l'issue d'un laps de temps prédéfini après le début du mode de remplissage ou de lavage, un état défectueux étant décelé lorsqu'aucune augmentation du poids n'est constatée à l'issue du laps de temps prédéfini.

14. Procédé selon la revendication 13, **caractérisé en ce que**, à l'issue d'un laps de temps prédéfini après le début du mode de remplissage ou de lavage, la valeur de la modification du poids du collecteur est déterminée dans un laps de temps prédéfini et la valeur de la modification du poids dans le laps de temps prédéfini est comparée à une valeur prédéfinie, un état défectueux étant décelé lorsque la valeur de la modification du poids dans le laps de temps prédéfini est inférieure à la valeur prédéfinie.

15. Procédé selon la revendication 13 ou la revendica-tion 14, **caractérisé en ce que** le poids du collec-teur est mesuré au début du mode de remplissage ou de lavage ou à l'issue du mode de remplissage ou de lavage.

16. Procédé selon la revendication 15, **caractérisé en ce que** le volume de liquide acheminé dans le sys-tème de liquide est calculé à partir de la différence de poids du collecteur au début du mode de remplissage ou de lavage et à l'issue du mode de remplissage et de lavage.

17. Procédé selon l'une quelconque des revendications 11 à 16, **caractérisé en ce qu'**une alarme acoustique et/ou optique est donnée après la constatation d'un état défectueux.

18. Procédé selon l'une quelconque des revendications 11 à 17, **caractérisé en ce que** le dispositif de traitement extracorporel du sang est un dispositif d'aphérèse, l'unité destinée à l'obtention d'un ou plusieurs constituants du sang étant une unité destinée à l'obtention de plasma, en particulier un filtre à plasma, et l'unité de traitement du sang une unité permettant de débarrasser le plasma de constituants pathogènes.
